# EUROPEAN PATENT APPLICATION

(11) **EP 2 923 707 A1**
(43) Date of publication of application: **30.09.2015**
(21) Application number: 14305453.4
(22) Date of filing: 28.03.2014
(51) Int. Cl.: A61K 38/17, A61K 38/38, A61K 38/41, A61K 38/42, A61K 31/375, A61K 31/70, A61K 33/06, A61K 33/10, A61K 33/14, A61P 7/00, A61P 7/04, A61P 7/08

(54) **Blood substitute with respiratory pigment**

(71) Applicant: Hemarina, 29600 Morlaix (FR)
(72) Inventor: Zal, M Franck., 29600 PLOUJEAN-MORLAIX (FR)
(74) Representative: Cabinet Plasseraud

(57) **Abstract**

The present invention relates to a composition comprising a respiratory pigment chosen from marine chlorocruorins, marine hemerythrins and marine hemocyanins, and a mixture of sodium chloride, calcium chloride, magnesium chloride or sulfate, potassium chloride, sodium gluconate and sodium acetate, for use as a blood substitute.

## Description

### FIELD OF THE INVENTION

The invention relates to a blood substitute comprising a respiratory pigment chosen from marine chlorocruorins, marine hemerythrins and marine hemocyanins, and a mixture of sodium chloride, calcium chloride, magnesium chloride or sulfate, potassium chloride, sodium gluconate and sodium acetate.

### BACKGROUND OF THE INVENTION

The present invention relates to a blood substitute comprising a respiratory pigment. One particular interest of such blood substitute is its use as oxygen carrier for industrial and therapeutic applications.

Two viable categories of oxygen therapeutics currently exist: haemoglobin-based oxygen carriers (HBOCs) and perfluorocarbons (PFCs). Haemoglobin taken directly from red blood cells can not be used as an intravascular oxygen carrier. To avoid spontaneous breakdown of haemoglobin and the toxicity of haemoglobin extracted from red blood cells, haemoglobin-based oxygen carriers use purified human (Riess et al. Chem Rev 2001, 101:2797), animal (bovine) (Lok et al Nature 2001, 410:855) or recombinant (Looker et al Nature 1992, 356:258) haemoglobin as raw materials. Each chain of purified haemoglobin is then covalently bridged with other globin chains or microencapsulated. Perfluorocarbons are liquid fluorinated hydrocarbon compounds capable of dissolving large amounts of oxygen and then delivering this oxygen. However, in this case the oxygen partial pressure needs to be higher than air oxygen partial pressure.

Oxygen therapeutics have been developed for two major purposes: i) they can function as alternatives to blood transfusion in order to avoid, reduce or delay transfusion of allogenic blood and ii) improvement of tissue oxygenation of organs with poor blood supply.

### SUMMARY OF THE INVENTION

One object of the invention is a composition comprising a respiratory pigment chosen from marine chlorocruorins, marine hemerythrins and marine hemocyanins, and a mixture of sodium chloride, calcium chloride, magnesium chloride or sulfate, potassium chloride, sodium gluconate and sodium acetate, for use as a blood substitute. Said composition is particularly useful for treating bleeding disorders.

An other object of the invention is the use of a composition comprising a respiratory pigment chosen from marine chlorocruorins, marine hemerythrins and marine hemocyanins, and a mixture of sodium chloride, calcium chloride, magnesium chloride or sulfate, potassium chloride, sodium gluconate and sodium acetate, as a blood substitute. Said use is particularly useful for the *in vitro* preservation of organs, cells and/or tissues.

Preferably, said composition is in the form of an aqueous solution, a frozen solution, a thawed solution or a lyophilised powder.

### DETAILED DESCRIPTION OF THE INVENTION

One object of the invention is a composition comprising a respiratory pigment chosen from marine chlorocruorins, marine hemerythrins and marine hemocyanins, and a mixture of sodium chloride, calcium chloride, magnesium chloride or sulfate, potassium chloride, sodium gluconate and sodium acetate, for use as a blood substitute. Said composition is particularly useful for treating bleeding disorders. Thus, the invention also aims to a composition according to the invention for use for treating bleeding disorders, preferably haemorrhages.

An other object of the invention is the use of a composition comprising a respiratory pigment chosen from marine chlorocruorins, marine hemerythrins and marine hemocyanins, and a mixture of sodium chloride, calcium chloride, magnesium chloride or sulfate, potassium chloride, sodium gluconate and sodium acetate, as a blood substitute. Said use is particularly useful for the *in vitro* preservation of organs, cells and/or tissues. Thus, the invention also aims to the use of a composition according to the invention for *in vitro* preserving organs and/or tissues and/or cells.

Preferably, the marine hemocyanins are chosen from hemocyanins from marine molluscs and hemocyanins from marine arthropods.
Marine hemocyanins are copper-containing proteins of a blue color. They usually have a molecular weight comprised between 300 000 to 9 000 000 Daltons.

Preferably, the marine chlorocruorins are chosen from chlorocruorins from marine polychaetes.

Marine chlorocruorins are iron-porphyrin-proteins of a green color. They usually have a molecular weight of around 2 750 000 Daltons.

Preferably, the marine hemerythrins are chosen from hemerythrins from sipunculids, priapulids, brachiopods or polychaetes.
Marine hemerythrins are iron proteins of a red/violet color. They usually have a molecular weight of around 108 000 Daltons.

Preferably, the respiratory pigment used in the composition of the invention is a marine hemerythrin from polychaetes.

The respiratory pigment used in the present invention may be a natural respiratory pigment, i.e. endogenously present in the marine organisms, but is can also be a recombinant version. By "recombinant", it is meant that said respiratory pigment is produced in a host cell, prokaryotic or eukaryotic, which has been genetically modified so as to express the respiratory pigment.

Preferably, the respiratory pigment is present said composition in an amount of 0,01 to 10 g/L, 0,2 to 0,6 g/L.

Moreover, the composition of the invention also comprises a mixture of sodium chloride, calcium chloride, magnesium chloride or sulfate, potassium chloride, sodium gluconate and sodium acetate.

Said composition may also comprise sugars, proteins, vitamins and antioxidants. Preferably, said composition also comprises at least a compound chosen from albumin, glucose, fructose, galactose, trehalose, sucrose, mannitol, sorbitol, glutathione and ascorbic acid.
The composition may also comprise a volume expander. Said volume expander may be chosen from saline solutions (for example saline solutions at 0.9% concentration), isotonic solutions like Ringer's lactate or Ringer's acetate, a dextrose solution (particularly at 5%), colloids, hydroxyethyl starch (also called hetastarch) like the products Hespan commercialized by B.Braun Medical Inc, Volulyte commercialized by Freseniux Kabi or Hextend® commercialized by BioTime Inc, and gelofusine.

The composition of the invention can be in the form of an aqueous solution, a thawed solution or a lyophilised powder. In one embodiment, the composition is conserved in a storage buffer. Said storage buffer is an acceptable medium. It may comprise 0-2.5 mM CaC12, 0-300 mM NaCl, 0-1 mM MgC12, 0-5 mM KCl, 0-1 M Hepes and/or Tris or any other buffer able to adjust the pH to the required value, adjusted to pH 7.0-7.8 with NaOH.

In one embodiment of the invention, the composition of the invention is formulated to have a pH of from about 6-8, preferably from about 7.0-7.8.

Another object of the invention is also a pouch comprising the composition of the invention.

Another object of the invention is also a method for improving cell growth, comprising the step of incubating a cell culture with a composition of the invention. Said cell culture may be chosen from eukaryotes, prokaryotes and cell lines. Said cell culture may be for example mammalian cells including human cells, fish cells, insect cells, mollusc cells, bacteria cells, annelids cells... Examples of types of cells to be cultured include, but are not limited to, kidney cells, hepatic cells, cardiac cells, pulmonary cells, intestinal cells, stomach cells, colon cells or pancreatic cells.

Another object of the invention is a method for improving recombinant protein production by host cells, comprising the step of incubating host cells with a composition of the invention. Said recombinant protein may be any protein of interest, for example an antibody such as an anti-TNFalpha antibody, an anti-IL-12 antibody, an anti-IL-18 antibody or an anti-EPO receptor antibody. Said host cells may be chosen from eukaryotes, prokaryotes and cell lines. Preferably, said types of cells are CHO (Chinese Hamster Ovary cell) or Sp2/0 or PTG2b cells which express a protein of interest such as an antibody.

The cell culture as described above or the host cells as described above may be incubated with a composition of the invention in the presence of a cell culture medium. Said cell culture medium may be Dulbecco's Modified Eagle's Medium (DMEM), DMEM/F12, Minimal Essential Medium (MEM), Basal Medium Eagle (BME), RPMI 1640, F-10, F-12, alpha-Minimal Essential Medium (alpha-MEM), Glasgow's Minimal Essential Medium (G-MEM), PF CHO (SAFC Biosciences), Ex-cell® 325 PF CHO serum-free medium for CHO cells protein-free (SAFC Bioscience) and Iscove's Modified Dulbecco's Medium, CD CHO medium.
Examples of cell culture medium also include the ones described in WO2008/033517. These cell culture mediums are serum-free cell culture mediums comprising Part A, Part B, and Part C, wherein
a) Part A consists essentially of a modified basal medium which excludes the following components: sodium bicarbonate, a buffer, mono-basic sodium phosphate, di-basic sodium phosphate, an osmolality regulator, a surfactant, and monosaccharide glucose;
b) Part B consists essentially of an inorganic iron source; and
c) Part C comprises a recombinant growth factor; a buffer; an osmolarity regulator; an energy source; and at least two different non-animal hydrolysates.
Preferably, such serum-free cell culture mediums comprise :
a) a modified basal medium which excludes the following components sodium bicarbonate, buffer, mono-basic sodium phosphate, di-basic sodium phosphate, an osmolality regulator, a surfactant, and monosaccharide glucose;
b) about 8 to 12 ml/kg or 122.45 mg/L ferric citrate; and
c) about 4 to 8 mL/kg or 10 to 14 mg/kg recombinant human insulin; about 5 to 9 g/kg anhydrous glucose; about 0.5 to 0.7 g/kg L-glutamine; about 1 to 2 g/kg sodium bicarbonate; about 1 to 2 g/kg HEPES; about 2 to 3 g/kg NaCl; about 0.5 to 2 g/kg surfactant (Pluronic F-68); about 0.01 to 0.1 g/kg NaH₂PO₄-H₂O; about 0.4 to 0.5 g/kg Na₂HPOWH₂O; about 8 to 12 g/kg yeast-based hydrolysate; and about 60 to 70 g/kg plant-based hydrolysate.

The invention also aims to the use of a composition according to the invention for *in vitro* preserving organs and/or tissues and/or cells. In said embodiment, the composition of the invention protects the cells and/or organs and/or tissues from cellular lesions induced by standard conservation. The composition of the invention can protect organs, tissues or cells from deleterious effects of ischemia reperfusion while maintaining metabolic needs of said organs, tissues or cells.

The use of a composition according to the invention for *in vitro* preserving organs and/or tissues and/or cells preferably relates to
- living tissues, such as skin, corneas, organs, such as, for example, heart, lung, kidney, liver or pancreas,
- organ parts, such as, for example, muscles, pancreatic islets, heart valves, and the like, and/or
- tissues or organs cells.
Such protection includes protection from damage caused by ischemia and/or anoxia during storage prior to transplantation in a subject.
For organ preservation, the organ (e.g. kidney) is removed from a donor (living, encephalic death or non-heart beating) and immediately thereafter infused with the composition of the invention. In the case of removal following death, the organ should be removed as soon as possible to prevent ischemic damage, generally within about 30 minutes to about 90 minutes. The organ is stored in excess composition of the invention at a temperature in the range of from about 2°C to about 12°C (hypothermic perfusion) preferably 4°C (cold ischemia) or in normothermic perfusion until later transplantation in a patient. Developments in the field of ex vivo organ preservation have advanced during the past quarter century to the point where organs for transplantation can be safely stored for variable periods depending upon the nature of the organ. Kidneys can be stored during one to two days (24-35 hours), livers and pancreases can be stored during less than one day (10-16/18 hours), but the clinically accepted limits for hearts is currently only about 6 hours or less. The composition of the invention provides protection of various organs for at least the maximum allowable storage time for each organ. Organs can be conserved in static or pulsative modes. In static mode the organ is bathed in the composition of the invention and in pulsative mode the organ is perfused using a machine comprising a pump system.

In addition to preservation of individual tissues or organs, the composition of the invention may also be used for whole body preservation of living donors, cadavers, including brain-dead individuals. In this manner preservation of the individual organs and other tissues for up to 8 hours or more can be achieved. The cadaver may be treated in substantially the same manner as described herein for bloodless hypothermic surgery, except that after the introduction of the composition of the invention at hypothermic temperature the individual is maintained under hypothermic temperature until such time as one or more organs are needed and are then harvested for use. The removed organs are then stored for transportation in additional fresh composition of the invention as needed.

The composition of the invention may also be used to preserve and protect tissues, e.g., skin, from storage damage and possibly from the harmful effects of toxins and chemical toxicants. Thus, the composition of the invention is also applicable to the storage of tissues, such as skin and corneas, for example, for later transplantation on, for example, burn patients, as well as to the protection of human skin against the harmful effects of toxins and toxicants, such as may be present in polluted environments, against chemical or germ warfare, and the like. For preservation of skin and corneal tissue for later transplantation, the tissue is removed from the donor and stored in composition of the invention for at least one week and up to about 2 to 4 weeks at 4°C until transplantation.

The invention also provides a method for preserving organs or tissues or cells from said organs or tissues, comprising:
- washing said organs or tissues or said cells from said organs or tissues after their collection, with the composition of the invention at a temperature from about 4°C to 37°C, preferably from about 4°C to 25°C, more preferably from about 4°C to 15°C,
- preserving said organs or tissues or said cells from said organs or tissues in static mode or dynamic perfusion at a temperature from about 4°C to 37°C, preferably from about 4°C to 25°C and more preferably from about 4°C to 15°C in the composition of the invention. The duration of the preservation will depend on the type of organs or tissues or cells as mentioned here above.

In one embodiment of the invention wherein the organs or tissues or cells are collected from a living or brain-dead subject, said organs or tissues or cells are washed and preserved preferably at 4°C.
In another embodiment of the invention wherein the organs or tissues or cells are collected from a subject who died from a cardiac arrest, said organs or tissues or cells are washed and preserved preferably at room temperature.

Another object of the invention is also an artificial oxygen carrier for transfusion comprising the composition of the invention.
As used herein, the term "artificial oxygen carrier for transfusion" refers to a biological product capable of replacing the haemoglobin present in the red blood corpuscles and capable of performing its function as a transporter of gas (oxygen and carbon dioxide). This artificial oxygen carrier for transfusion also has to supply oxygen to the tissues, where it becomes charged with CO2, to release this gas at the exchange surfaces (lungs). This artificial oxygen carrier for transfusion may also be called blood substitute.
The artificial oxygen carrier for transfusion of the invention presents the following advantages: it is not toxic, it has no pathogenic agent, it is transfusable into all blood types, it has a sufficiently long residence time to ensure regeneration into natural haemoglobin of the organism into which it is transfused and it is eliminated by the organism into which it is transfused.
The expression "non-toxic" means that the artificial oxygen carrier for transfusion does not cause any pathological disorder of an immune-reaction, allergic or nephrotoxic type. The expression "has no pathogenic agent" refers to the absence of identified microorganisms or viruses. The absence of pathological disorders indirectly implies the absence of pathogens. The expression "transfusable into all blood types" refers to the absence of blood typing (ABO or rhesus system). The expression "has a sufficiently long residence time to ensure regeneration into natural haemoglobin of the organism into which it is transfused" refers to the time which is long enough to enable an organism to synthesise back its own red blood cells. By way of illustration, within the framework of the transfusion of a human being, the time must advantageously be of the order of 48 hours.

Advantageously, the artificial oxygen carrier for transfusion of the invention is pyrogen-free and microbe-free.

### EXAMPLE: Extraction of hemerythrin usable as blood substitute

As an example, hemerythrin according to the invention is obtained by grinding marine worms, like the marine polychaetes *Nereis diversicolor.* After cold centrifugation of the grinded mixture (15 000 g for 15 min at 4°C) to eliminate any tissue debris, the supernatants are frozen at -20° C or in liquid nitrogen, or immediately purified.
Before purification, the thawed sample is centrifuged, at 5 000 g for 5 min at 4° C. After centrifugation, a small residue is generally present; this is eliminated.
The supernatant is then purified using a Superose 6-C or Sepharose column. The samples are eluted with a buffer having the following composition, for one litre: 23.38 g NaCl (400 mM); 0.22 g KCl (2.95 mM); 7.88 g MgSO₄, 7H₂O (31.97 mM); 1.62 g CaCl₂, 2H₂O (11.02 mM) and HEPES (50 mM). The pH is adjusted to pH=7.0 by adding HCl. The rate used is generally 0.4 to 0.5 ml/min.

## Claims

1. Composition comprising a respiratory pigment chosen from marine chlorocruorins, marine hemerythrins and marine hemocyanins, and a mixture of sodium chloride, calcium chloride, magnesium chloride or sulfate, potassium chloride, sodium gluconate and sodium acetate, for use as a blood substitute.

2. Use of a composition comprising a respiratory pigment chosen from marine chlorocruorins, marine hemerythrins and marine hemocyanins, and a mixture of sodium chloride, calcium chloride, magnesium chloride or sulfate, potassium chloride, sodium gluconate and sodium acetate, as a blood substitute.

3. Composition or use according to claim **1 or 2**, wherein the marine hemocyanins are chosen from hemocyanins from marine molluscs and hemocyanins from marine arthropods.

4. Composition or use according to any one of claims **1 to 3**, wherein the marine chlorocruorins are chosen from chlorocruorins from marine polychaetes.

5. Composition or use according to any one of claims **1 to 4**, wherein the marine hemerythrins are chosen from hemerythrins from sipunculids, priapulids, brachiopods or polychaetes.

6. Composition or use according to any one of claims **1 to 5**, wherein it also comprises at least a compound chosen from albumin, glucose, fructose, galactose, trehalose, sucrose, mannitol, sorbitol, glutathione and ascorbic acid.

7. Composition or use according to any of claims **1 to 6**, wherein said composition is in the form of an aqueous solution, a thawed solution or a lyophilised powder.

8. Composition or use according to any of claims **1 to 7,** wherein the respiratory pigment is present said composition in an amount of 0.5 to 10 g/L.

9. Composition according to any of claims **1 or 3 to 8,** for use for treating bleeding disorders, preferably haemorrhages.

10. Use of a composition according to any of claims **2 to 8,** for *in vitro* preserving organs and/or tissues and/or cells.

11. Pouch comprising a composition according to claim **1**.

12. Method for preserving organs or tissues or cells, comprising:
- washing said organs or tissues or cells, with the composition according to claim **1** at a temperature from about 4°C to 37°C, preferably from about 4°C to 25°C, more preferably from about 4°C to 15°C,
- preserving said organs or tissues or cells in static mode or dynamic perfusion at a temperature from about 4°C to 37°C, preferably from about 4°C to 25°C and more preferably from about 4°C to 15°C in said composition.
